# EUROPÄISCHE PATENTSCHRIFT

(11) **EP 2 272 556 B1**
(45) Veröffentlichungstag und Bekanntmachung des Hinweises auf die Patenterteilung: **21.12.2011**
(21) Anmeldenummer: 09164638.0
(22) Anmeldetag: 06.07.2009
(51) Int. Cl.: A61M 11/00, F04B 39/06, F04B 49/10, A61M 16/00

(54) **Kompressor einer Aerosoltherapievorrichtung**
Compressor for an aerosol therapy device
Compresseur d'un dispositif de thérapie par aérosol

(43) Veröffentlichungstag der Anmeldung: 12.01.2011
(73) Patentinhaber: PARI GmbH Spezialisten für effektive Inhalation, 82319 Starnberg (DE)
(72) Erfinder: Zemke, Robert, 80339 München (DE)
(74) Vertreter: Siegert, Georg

(56) Entgegenhaltungen:
- WO-A-2005/013879
- WO-A-2007/048206
- DE-A1-102006 012 174
- DE-B3- 10 239 321
- US-A1- 2005 112 013
- US-A1- 2005 166 921

## Beschreibung

Die vorliegende Erfindung betrifft einen Kompressor einer Aerosoltherapievorrichtung mit einem Vernebler zum Vernebeln eines Mediums, insbesondere eines flüssigen Mediums, mit den Merkmalen des Oberbegriffs von Anspruch 1.

Derartige Kompressoren werden beispielsweise dazu genutzt, um dem Vernebler unter Druck/Gas, in der Regel Druckluft, zuzuführen und dadurch ein therapeutisch wirksames Fluid zu vernebeln. Beispielsweise ist aus der EP 0 170 715 A eine Aerosoltherapievorrichtung bekannt, bei der ein Medium über eine Druckmittel betriebene Verneblerdüse, der das Druckmittel und das Medium zugeführt wird, vernebelt wird (Düsenvernebler). Dabei beschreibt die EP 0 170 715 A eine zerstäubervorrichtung, bei der der Anwender das erzeugte Aerosol über ein Mundstück einatmet. Daneben ist bekannt, für eine Behandlung der Nasenhöhle das erzeugte Aerosol direkt der Nase eines Anwenders zuzuführen, wobei die DE 102 39 321 B darüber hinaus erläutert, der durch das Druckmittel erzeugten und durch die Nase strömenden Aerosolgrundströmung zusätzlich Druckschwankungen zu überlagern, um ein Eindringen des Aerosols in die Nasennebenhöhlen zu erreichen bzw. zu unterstützen. Dafür sind zwei Kompressoren erforderlich, nämlich ein Kompressor für die Erzeugung der Druckluft und ein Kompressor für die Erzeugung der Druckschwankungen. Beide Kompressoren wirken derart zusammen, dass mit Hilfe der eine Verneblerdüse umfassenden Aerosoltherapievorrichtung eine Aerosolgrundströmung mit überlagerter Druckschwankung erzeugt wird. Alternativ ist es aus der DE 10 2006 012 174 A bekannt statt zweier Kompressoren nur einen Kompressor mit zwei Kompressionsräumen zu verwenden. Auch sind Aerosoltherapievorrichtungen denkbar, bei denen der Kompressor nur zur Überlagerung einer Aerosolströmung die z. B. durch das Einatmen eines Anwenders erzeugt wird, mit einer Druckschwankung genutzt wird. Dieses Prinzip kann insbesondere bei Membranverneblern zum Einsatz kommen, die zur Verneblung des Mediums keine Grundströmung erfordern.

Als Kompressoren werden bei Aerosoltherapievorrichtungen mit Verneblerdüse gängigerweise elektromotorisch angetriebene Kolben oder Membrankompressoren eingesetzt, wie sie z. B. in der DE 199 27 528 A oder in der DE 102 39 321 A beschrieben sind. Es ist aber auch der Einsatz anderer Kompressoren denkbar.

Die Kompressoren solcher Aerosoltherapievorrichtungen sind in der Regel in einem Gehäuse aufgenommen, mit dem der Vernebler, sei es ein Düsen- oder ein Membranvernebler zu verbinden ist. Derartige Kompressoren müssen ferner der Richtlinie IEC60601-1:2005 genügen, die maximale Grifftemperaturen an der Außenseite des Gehäuses festlegt. Während des Betriebs erwärmen sich die Kompressoren bzw. die Kompressorbaugruppe insbesondere durch die Verlustleistung der verwendeten Elektromotoren und die Kompressionswärme. Insbesondere bei kleinen, kompakten Bauformen und bei Geräten mit geschlossenen Gehäusen kann dieser Wärmeeintrag dazu führen, dass die zulässigen Grifftemperaturen an der Außenseite des Gehäuses, die in der Richtlinie IEC60601-1:2005 definiert sind, nicht eingehalten werden können. Andererseits ist eine Verwendung von Lüftern zur Reduktion dieser Grifftemperaturen bei solchen Geräten technisch nicht praktikabel oder aus verschiedenen Gründen nicht sinnvoll.

Ferner ist ein Kompressor mit den Merkmalen im Oberbegriff des Anspruchs 1 aus der WO 2005/013879 A oder der US 2005/166921 A bekannt. Die WO 2007/048206 A offenbart ferner eine Beatmungsvorrichtung mit einem Gebläse, das in einem Außengehäuse aufgenommen ist.

Daher beruht die Aufgabe der vorliegenden Erfindung darin einen Kompressor einer Aerosoltherapievorrichtung der eingangs genannten Art derart weiterzubilden, dass durch passive Kühlmaßnahmen die maximal zulässigen Grifftemperaturen gemäß IEC60601-1:2005 auf technisch praktikable und sinnvolle Weise eingehalten werden können.

Diese Aufgabe wird durch einen Kompressor mit den Merkmalen des Anspruchs 1 gelöst. Vorteilhafte Weiterbildungen sind den Unteransprüchen zu entnehmen.

Der vorliegenden Erfindung liegt der Gedanke zu Grunde das Gehäuse der eingangs beschriebenen Kompressoren als doppelwandiges Gehäuse auszugestalten. Dadurch ist eine äußere Gehäuseschale über einen Spalt in einem Abstand zu einer inneren Gehäuseschale angeordnet und eine Luftschicht zwischen diesen Gehäuseschalen dient der Isolation. Dabei kann über den Abstand der beiden Gehäuseschalen der gewünschte Kühleffekt eingestellt werden. Vorteilhafterweise ist der Spalt beidseits seitlich des Gehäuses offen sein und ermöglicht so einen Luftstrom zwischen den beiden Gehäuseschalen, der zur weiteren Wärmeabfuhr dient. Der Luftstrom wird durch Konvektion ausgehend von der wärmeren inneren Gehäuseschale erzeugt und hängt zusätzlich von der Lage des Geräts ab.

Dementsprechend kennzeichnet sich ein Kompressor einer Aerosoltherapievorrichtung gemäß der vorliegenden Erfindung gegenüber den eingangs genannten Kompressoren dadurch, dass das Gehäuse einen äußeren Gehäuseteil aufweist, der den inneren Gehäuseteil zur Bildung einer Luftschicht zwischen dem inneren und äußeren Gehäuseteil in einem Abstand und zumindest teilweise umgibt. Durch die Luftschicht wird zwischen dem inneren Gehäuseteil und dem äußeren Gehäuseteil eine Isolationsschicht gebildet. Dadurch wird die Grifftemperatur, d. h. die Oberflächentemperatur des äußeren Gehäuseteils, reduziert.

Vorteilhafterweise kommt dieses Prinzip zum Einsatz, wenn der innere Gehäuseteil die Kompressorbaugruppe im Wesentlichen vollständig umschließt, so dass eine Verschmutzung der Kompressorbaugruppe und der Kontakt des Anwenders mit Strom führenden oder bewegten Teilen der Kompressorbaugruppe vermieden wird. Insbesondere bei derartigen kompakten Bauformen mit geschlossenen Gehäusen ist eine Maßnahme zur Reduzierung der Grifftemperaturen erforderlich. Dabei hat der innere Gehäuseteil je nachdem ob ein oder mehrere Kompressorbaugruppen vorgesehen sind höchstens je zwei Ansaugöffnungen und zwei Ausgabeöffnungen zu und von der jeweiligen Kompressorbaugruppe und ist ansonsten vollständig geschlossen. Kommt eine Kompressorbaugruppe zum Einsatz, bei der ausschließlich eine Fluidströmung zu erzeugen ist, ohne dass die Aerosolgrundströmung mit einer Druckschwankung überlagert wird, wie es in der Beschreibungseinleitung erwähnt ist, so weist die Kompressorbaugruppe nur eine Ansaug- und eine Ausgabeöffnung auf. Soll darüber hinaus auch eine überlagernde Druckschwankung vorgesehen werden, so werden entweder zwei Kompressorbaugruppen mit jeweils einer Ansaug- und einer Ausgabeöffnung vorgesehen, von denen eine der Fluidströmung und die andere der Druckschwankungsüberlagerung dient oder aber eine Kompressorbaugruppe, wie sie in der DE 10 2006 012 174 beschrieben ist, wobei die Kompressorbaugruppe zwei Ansaug- und Ausgabeöffnungen aufweist. Hier kann ggf. bei einer besonderen Ausführungsform die der Druckschwankung dienende Ansaug- und Ausgabeöffnung durch nur eine Öffnung gebildet werden, so dass diese Kompressorbaugruppe nur drei Öffnungen aufweist. Diesbezüglich wird der Fachmann auf die DE 10 2006 012 174 bezüglich weiterer Informationen verwiesen. Ferner kann hier der innere Gehäuseteil das Kompressorgehäuse sein, der bevorzugt neben der/den Kompressorbaugruppe (-n) eine Elektronik und/oder ein Netzteil zur Steuerung der Kompressorbaugruppe(-n) bzw. zur Stromversorgung derselben beinhaltet.

Um die von dem inneren Gehäuseteil abgegebene Wärme kontinuierlich abtransportieren zu können, weist der äußere Gehäuseteil an gegenüberliegenden Seiten (vertikal oder horizontal) je wenigstens eine Öffnung auf, so dass die Luftschicht mit der Umgebung kommunizieren und ein Luftaustausch stattfinden kann bzw. ein Luftstrom entsteht. Der Luftstrom entsteht dabei durch Konvektion und kann durch eine vertikale Ausrichtung der Öffnungen nach Art eines Kamineffekts verstärkt werden. Durch den ständigen Austausch der Luft zwischen den beiden Gehäuseteilen liegt deren Temperatur zu jeder Zeit weit unter der Temperatur des inneren Gehäuseteils. Ferner ist durch den äußeren Gehäuseteil der Kontakt des Anwenders mit dem wärmeren inneren Gehäuse ausgeschlossen.

Als besonders vorteilhaft hat sich diesbezüglich herausgestellt, wenn der äußere Gehäuseteil den inneren Gehäuseteil um eine Achse (vertikal oder horizontal) vollständig umgibt und an gegenüberliegenden Enden der Achse zumindest im Bereich der Luftschicht offen ist.

Darüber hinaus hat sich als praktikables Maß für den Abstand zwischen dem inneren und dem äußeren Gehäuseteil ein Abstand von wenigstens 1 mm, vorzugsweise wenigstens 2 mm und am meisten bevorzugt wenigstens 3 mm herausgestellt. Bevorzugterweise sollte dieser Abstand weniger als 30 mm, bevorzugterweise weniger als 20 mm und am meisten bevorzugt weniger als 10 mm betragen.

Darüber hinaus ist es aus fertigungstechnischen Gründen bevorzugt, dass der äußere Gehäuseteil und der innere Gehäuseteil durch ein doppelwandiges Gehäuse einstückig ausgebildet sind, wobei die innen liegende Wand den inneren Gehäuseteil und die außen liegende Wand den äußeren Gehäuseteil bilden. Diesbezüglich versteht sich, dass nicht das gesamte Gehäuse einstückig ausgebildet sein muss, sondern das Gehäuse aus innerem und äußerem Gehäuseteil auch mehrschalig, z. B. aus zwei Gehäusehälften zusammengesetzt sein kann, dennoch aber äußere und innere Gehäuseteile einstückig miteinander ausgebildet sind.

Schließlich schlägt die vorliegende Erfindung auch eine Aerosoltherapievorrichtung mit einem Vernebler, sei es ein Düsen- oder ein Membranvernebler (siehe Einleitung), zum Vernebeln eines Mediums, insbesondere eines flüssigen Mediums und einem Kompressor, der vorstehenden Art vor.

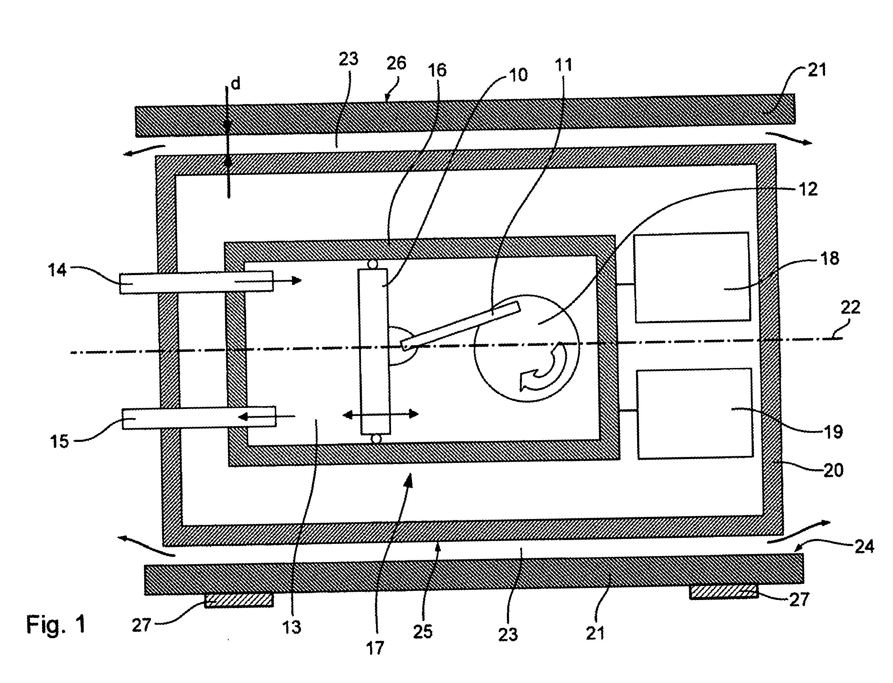

Weitere Vorteile und Merkmale der vorliegenden Erfindung sind aus der folgenden Beschreibung einer bevorzugten Ausführungsform, die anhand der begleitenden Figur 1 erfolgt, ersichtlich, wobei:
Figur 1 eine schematische Querschnittsansicht eines erfindungsgemäßen Kompressors zeigt.

Bei dem in Figur 1 dargestellten Kompressor handelt es sich um einen Hubkolbenkompressor. Die Kompressorbaugruppe 17 umfasst hierfür einen Kolben 10, der über einen Pleuel 11 und einen Exzenter 12 in einer Kompressionskammer 13, angetrieben durch einen nicht dargestellten Elektromotor, hin und her bewegbar ist. In den Kompressionsraum 13 mündet eine Ansaugöffnung 14, die mit der Atmosphäre (Umgebung) in Verbindung steht sowie eine Ausgabeöffnung 15, die vorzugsweise über einen Schlauch mit einem nicht dargestellten Vernebler, z. B. einem Düsenvernebler oder einem Membranvernebler, einer nicht dargestellten Aerosoltherapievorrichtung verbindbar ist, um z. B. die eingangs genannte Druckluft als Druckmittel eines Düsenverneblers zur Verneblung des Mediums am Vernebler bereitzustellen. Die Kompressorbaugruppe 17 ist in einem Kompressorgehäuse 16 aufgenommen, das in einem Gehäuse aufgenommen ist. Das Gehäuse setzt sich aus einem inneren Gehäuseteil 20 und einem äußeren Gehäuseteil 21 zusammen. Der innere Gehäuseteil 20 beinhaltet ferner eine schematisch dargestellte Steuerung 18 zur Steuerung der Kompressorbaugruppe 17. Ferner ist ein Netzteil 19 vorgesehen, das über einen nicht dargestellten Netzanschluss verfügt und der Stromversorgung von Steuerung 18 und Kompressorbaugruppe 17 dient.

Der äußere Gehäuseteil 21 umgibt den inneren Gehäuseteil 20 um eine hier horizontale Achse 22 vollständig. Zwischen dem inneren Gehäuseteil 20 und dem äußeren Gehäuseteil 21 verbleibt jedoch ein Spalt 23, in dem eine Luftschicht gebildet ist. Da kein großflächiger Kontakt zwischen der Innenfläche 24 des äußeren Gehäuseteils 21 und der Außenfläche 25 des inneren Gehäuseteils 20 besteht, erfolgt eine Erwärmung des äußeren Gehäuseteils 21 ausschließlich oder im Wesentlichen ausschließlich durch Wärmestrahlung von der Außenfläche 25 des inneren Gehäuseteils 20 über die Luftschicht im Spalt 23 zum äußeren Gehäuseteil 21. Dabei wirkt die Luftschicht im Spalt 23 als Isolationsschicht und reduziert die Erwärmung des äußeren Gehäuseteils 21 und damit dessen Außenfläche 26. Die Außenfläche 26 kann damit den maximal zulässigen Grifftemperaturen gemäß der Richtlinie IEC60601-1:2005 genügen.

Ferner ist der äußere Gehäuseteil 21 an gegenüberliegenden Enden der Achse 22 zumindest im Bereich der Luftschicht im Spalt 23 offen. Durch Konvektion, die durch die wärmere Außenfläche 25 des inneren Gehäuseteils 20 gegenüber der Innenfläche 24 des äußeren Gehäuseteils 21 entsteht, bildet sich ein Luftstrom, in dem die Luftschicht enthaltenden Spalt 23, so dass die von der Außenfläche 25 des inneren Gehäuseteils 20 abgestrahlte Wärme nach außen abtransportiert wird, wie es durch die Pfeile in Figur 1 dargestellt ist.

Des Weiteren kann der Abstand d des Spalts 23, in dem die Luftschicht gebildet ist, entsprechend eingestellt werden, um die Grifftemperatur der Außenfläche 26 des äußeren Gehäuseteils 21 einzustellen. Bevorzugterweise liegt dieser Spalt bei wenigstens 1 mm und vorzugsweise wenigstens 2 mm und am meisten bevorzugt wenigstens 3 mm und unter 30 mm bzw. 20 mm und am meisten bevorzugt 10 mm.

Darüber hinaus weist der äußere Gehäuseteil 21 Füße 27 auf, die dazu dienen den Kompressor auf einer Fläche abzustellen. Ferner ist es denkbar, dass der äußere Gehäuseteil 21 eine Handhabe aufweist, die vorzugsweise aus dem gleichen Material angeformt oder befestigt ist und dem manuellen Transport des Kompressors dient. Die Handhabe ist in Figur 1 jedoch nicht dargestellt. Auch ist es denkbar die Füße 27 alternativ oder zusätzlich an der in Figur 1 rechten Seite vorzusehen, so dass das Gehäuse vertikal aufgestellt werden kann. Dadurch wird ein Kamineffekt im Spalt 23 mit einem erhöhten Luftaustausch erzielt.

Ferner ist der innere Gehäuseteil 20 so ausgestaltet, dass er die Kompressorbaugruppe 17 vollständig umgibt und nur die Ansaugöffnung 14 und die Ausgabeöffnung 15 umfasst, ansonsten bis auf ggf. einen Netzanschluss des Netzteils 19 aber vollständig geschlossen ausgestaltet ist.

Alternativ wäre es auch denkbar einen Kompressor zu verwenden, wie er in der DE 10 2006 012 174 A beschrieben ist. In diesem Fall hätte der innere Gehäuseteil 20 eine zusätzliche dritte Öffnung, die gleichzeitig als Aus- wie auch Eingabeöffnung dient oder aber vier Öffnungen, von denen zwei als Ansaugöffnung und zwei als Ausgabeöffnung dienen. Letzteres wäre auch der Fall, wenn der innere Gehäuseteil 20 zwei Kompressorbaugruppen 17 enthielte.

In einem ersten Versuch konnte der Effekt der vorliegenden Erfindung bereits erfolgreich nachgewiesen werden. Dabei wurde ein Heizkörper in einem vollständig geschlossenen, inneren Gehäuse erwärmt und die Temperatur des inneren Gehäuses an der Außenfläche oberhalb des Heizelements gemessen. Der Luftspalt bzw. die Luftschicht zwischen dem inneren Gehäuse und dem äußeren Gehäuse wurde auf ca. 3 mm festgelegt und die Temperatur an der Außenfläche des Außengehäuses an der gleichen Stelle wie am Innengehäuse oberhalb des Heizelements gemessen. Dabei wurde festgestellt, dass die Grifftemperatur der Außenfläche des äußeren Gehäuses um bis zu 30°C kühler war als die des inneren Gehäuses. Dabei stieg der Kühleffekt mit zunehmender Temperatur des inneren Gehäuses an, wobei die Lufttemperatur im Spalt maximal 9°C über der Umgebungstemperatur lag.

Die Ausgestaltung des Kompressors der vorliegenden Erfindung hat sich damit als besonders vorteilhaft herausgestellt, da zum einen die Grifftemperatur des äußeren Gehäuseteils 21 und damit des Gehäuseteils, mit dem der Anwender in Kontakt kommt, erheblich reduziert werden konnte, darüber hinaus aber die aus dem Spalt abgeführte Luft nur geringfügig über der Außentemperatur bzw. Umgebungstemperatur lag, so dass auch von ausströmender Luft keine Gefahr ausgeht. Damit stellt das erfinderische Konzept der vorliegenden Erfindung auf einfachste Art und Weise eine einfach in die Praxis umsetzbare passive Kühlung des Gehäuses bereit, die darüber hinaus kostengünstig umsetzbar ist.

## Patentansprüche

1. Kompressor einer Aerosoltherapievorrichtung mit einem Vernebler zum Vernebeln eines Mediums umfassend:
ein Gehäuse mit dem der Vernebler zu verbinden ist, und
wenigstens eine Kompressorbaugruppe (17) zum Erzeugen einer Fluidströmung, wobei das Gehäuse einen inneren Gehäuseteil (20) aufweist, in dem die Kompressorbaugruppe (17) aufgenommen ist, wobei das Gehäuse einen äußeren Gehäuseteil (21) aufweist, der den inneren Gehäuseteil (20) zur Bildung einer Luftschicht zwischen dem inneren (20) und äußeren (21) Gehäuseteil in einem Abstand (d), zumindest teilweise umgibt,
**dadurch gekennzeichnet, dass**
der äußere Gehäuseteil (21) an gegenüberliegenden Seiten je wenigstens eine Öffnung aufweist, so dass die Luftschicht mit der Umgebung in Verbindung steht und durch Konvektion ausgehend von dem wärmeren inneren Gehäuseteil ein Luftstrom erzeugt wird.

2. Kompressor nach Anspruch 1, bei der der innere Gehäuseteil (20) höchstens je zwei Ansaugöffnungen (14) und zwei Ausgabeöffnungen (15) zu und von der Kompressorgruppe (17) aufweist und ansonsten vollständig geschlossen ist.

3. Kompressor nach Anspruch 1 oder 2, bei dem der äußere Gehäuseteil (21) den inneren Gehäuseteil (20) um eine Achse (22) vollständig umgibt und an gegenüberliegenden Enden der Achse (22) zumindest im Bereich der Luftschicht offen ist.

4. Kompressor nach einem der vorstehenden Ansprüche, bei dem der Abstand (d) zwischen dem inneren (20) und dem äußeren (21) Gehäuseteil wenigstens 1 mm beträgt.

5. Kompressor nach Anspruch 4, bei dem der Abstand (d) zwischen dem inneren (20) und dem äußeren (21) Gehäuseteil wenigstens 2mm beträgt.

6. Kompressor nach Anspruch 5, bei dem der Abstand (d) zwischen dem inneren (20) und dem äußeren (21) Gehäuseteil wenigstens 3 mm beträgt.

7. Kompressor nach einem der vorstehenden Ansprüche, bei dem der äußere Gehäuseteil (21) und der innere Gehäuseteil (20) durch ein doppelwandiges Gehäuse einstückig ausgebildet sind, wobei die innen liegende Wand den inneren Gehäuseteil und die außen liegende Wand den äußeren Gehäuseteil bilden.

8. Kompressor nach Anspruch 7, bei dem das doppelwandige Gehäuse teilbar ausgebildet ist.

9. Aerosoltherapievorrichtung mit einem Vernebler zum Vernebeln eines Mediums und einem Kompressor nach einem der vorstehenden Ansprüche.

## Claims

1. Compressor of an aerosol treatment device having a nebuliser for nebulising a medium comprising:
a housing to which the nebuliser is to be connected, and at least one compressor assembly (17) for generating a fluid stream, wherein the housing has an inner housing part (20), in which the compressor assembly (17) is accommodated, wherein the housing has an outer housing part (21) which at least partly surrounds the inner housing part (20) to form an air layer between the inner (20) and outer (21) housing part at a distance (d), **characterised in that** the outer housing part (21) has in each case at least one opening on opposite sides so that the air layer is connected to the surroundings and an air stream is generated by convection starting from the warmer inner housing part.

2. Compressor according to claim 1, in which the inner housing part (20) has at the most in each case two intake openings (14) and two output openings (15) to and from the compressor group (17) and otherwise is completely closed.

3. Compressor according to claim 1 or 2, in which the outer housing part (21) completely surrounds the inner housing part (20) about an axis (22) and at opposite ends of the axis (22) is open at least in the region of the air layer.

4. Compressor according to one of the above claims, in which the distance (d) between the inner (20) and the outer (21) housing part is at least 1 mm.

5. Compressor according to claim 4, in which the distance (d) between the inner (20) and the outer (21) housing part is at least 2 mm.

6. Compressor according to claim 5, in which the distance (d) between the inner (20) and the outer (21) housing part is at least 3 mm.

7. Compressor according to one of the above claims, in which the outer housing part (21) and the inner housing part (20) are designed to be integral due to a double-walled housing, wherein the inner-lying wall forms the inner housing part and the outer-lying wall forms the outer housing part.

8. Compressor according to claim 7, in which the double-walled housing is designed to be separable.

9. Aerosol treatment device having a nebuliser for nebulising a medium and a compressor according to one of the above claims.

## Revendications

1. Compresseur d'un dispositif de thérapie par aérosol avec un pulvérisateur pour la pulvérisation d'un fluide, comprenant :
un boîtier, auquel le pulvérisateur doit être raccordé, et
au moins un ensemble de compression (17) pour la génération d'un écoulement fluidique, le boîtier présentant une partie de boîtier intérieure (20) recevant l'ensemble de compression (17), le boîtier présentant une partie de boîtier extérieure (21) qui entoure au moins en partie la partie de boîtier intérieure (20) pour la formation d'une couche d'air entre les parties de boîtier intérieure (20) et extérieure (21) à une distance (d),
**caractérisé en ce que**
la partie de boîtier extérieure (21) présente respectivement au moins une ouverture sur des côtés opposés de sorte que la couche d'air soit en liaison avec l'environnement et un courant d'air soit généré par convection en partant de la partie de boîtier intérieure plus chaude.

2. Compresseur selon la revendication 1, pour lequel la partie de boîtier intérieure (20) présente au maximum respectivement deux ouvertures d'aspiration (14) et deux ouvertures de sortie (15) vers et depuis le groupe de compression (17) et sinon est fermée complètement.

3. Compresseur selon la revendication 1 ou 2, pour lequel la partie de boîtier extérieure (21) entoure complètement la partie de boîtier intérieure (20) autour d'un axe (22) et est ouverte sur des extrémités opposées de l'axe (22) au moins dans la zone de la couche d'air.

4. Compresseur selon l'une quelconque des revendications précédentes, pour lequel la distance (d) entre la partie de boîtier intérieure (20) et la partie de boîtier extérieure (21) s'élève au moins à 1 mm.

5. Compresseur selon la revendication 4, pour lequel la distance (d) entre la partie de boîtier intérieure (20) et la partie de boîtier extérieure (21) s'élève au moins à 2 mm.

6. Compresseur selon la revendication 5, pour lequel la distance (d) entre la partie de boîtier intérieure (20) et la partie de boîtier extérieure (21) s'élève au moins à 3 mm.

7. Compresseur selon l'une quelconque des revendications précédentes, pour lequel la partie de boîtier extérieure (21) et la partie de boîtier intérieure (20) sont réalisées d'un seul tenant par un boîtier à double paroi, la paroi s'étendant à l'intérieur formant la partie de boîtier intérieure et la paroi s'étendant à l'extérieur formant la partie de boîtier extérieure.

8. Compresseur selon la revendication 7, pour lequel le boîtier à double paroi est réalisé de manière sécable.

9. Dispositif de thérapie par aérosol avec un pulvérisateur pour la pulvérisation d'un fluide et un compresseur selon l'une quelconque des revendications précédentes.
